(19) Europäisches Patentamt / European Patent Office / Office européen des brevets

(11) EP 2 797 612 B1

(12) FASCICULE DE BREVET EUROPEEN

(45) Date de publication et mention de la délivrance du brevet:
**20.01.2021 Bulletin 2021/03**

(21) Numéro de dépôt: **12808833.3**

(22) Date de dépôt: **26.12.2012**

(51) Int Cl.:
***A61K 36/54*** (2006.01) ***C11B 1/04*** (2006.01)
***C11B 1/06*** (2006.01) ***C11B 1/10*** (2006.01)
***C11B 3/12*** (2006.01) ***A61P 19/02*** (2006.01)
***A61P 19/04*** (2006.01)

(86) Numéro de dépôt international:
**PCT/EP2012/076903**

(87) Numéro de publication internationale:
**WO 2013/098293 (04.07.2013 Gazette 2013/27)**

# (54) UTILISATION D'AVOCATS MOUS ENTIERS POUR OBTENIR UNE HUILE D'AVOCAT RICHE EN INSAPONIFIABLE

VERWENDUNG VON GANZEN WEICHEN AVOCADOS ZUR HERSTELLUNG VON AVOCADOÖL REICH AN UNVERSEIFBAREN

USE OF WHOLE SOFT AVOCADOS TO OBTAIN AVOCADO OIL RICH IN UNSAPONIFIABLE MATTER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.12.2011 FR 1162461**

(43) Date de publication de la demande:
**05.11.2014 Bulletin 2014/45**

(73) Titulaire: **Laboratoires Expanscience**
**92048 Paris La Défense Cedex (FR)**

(72) Inventeurs:
- **MSIKA, Philippe**
  **F-78000 Versailles (FR)**
- **LEGRAND, Jacques**
  **F-61290 Neuilly Sur Eure (FR)**

(74) Mandataire: **Regimbeau**
**20, rue de Chazelles**
**75847 Paris Cedex 17 (FR)**

(56) Documents cités:
**WO-A2-2004/012496 WO-A2-2010/026595**
**FR-A1- 2 798 667**

- **anonymous: "ASU Expanscience - Cutting Edge Technology to produce a unique and original ASU", , 13 février 2010 (2010-02-13), pages 1-5, XP002674953, Extrait de l'Internet: URL:http://web.archive.org/web/20100213004455/http://www.original-asu.com/Cutting_Edge__Technology.html [extrait le 2012-04-25]**
- **M. J. WERMAN ET AL: "Avocado oil production and chemical characteristics", JOURNAL OF THE AMERICAN OIL CHEMISTS' SOCIETY, vol. 64, no. 2, 1 février 1987 (1987-02-01), pages 229-232, XP055025527, ISSN: 0003-021X, DOI: 10.1007/BF02542007**
- **"Nahrungsfette und Öle - [Extract] ED - BOCKISCH M", 1 janvier 1993 (1993-01-01), NAHRUNGSFETTE UND -ÖLE, STUTTGART, ULMER, DE, PAGE(S) 73,82,175 - 177, XP009158090, ISBN: 978-3-8001-5817-1 le document en entier**
- **KASHMAN Y ET AL: "New compounds from avocado pear", TETRAHEDRON, ELSEVIER SCIENCE PUBLISHERS, AMSTERDAM, NL, vol. 25, no. 18, 1 janvier 1969 (1969-01-01), pages 4617-4631, XP002090295, ISSN: 0040-4020, DOI: 10.1016/S0040-4020(01)83005-2**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- BRIAN I. BROWN: "Isolation of unpleasant flavor compounds in the avocado (Persea americana)", JOURNAL OF AGRICULTURAL AND FOOD CHEMISTRY, vol. 20, no. 4, 1 juillet 1972 (1972-07-01), pages 753-757, XP055025721, ISSN: 0021-8561, DOI: 10.1021/jf60182a019
- GUTFINGER T ET AL: "Studies of unsaponifiables in several vegetable oils", LIPIDS, SPRINGER, US, vol. 9, no. 9, 1 janvier 1974 (1974-01-01) , pages 658-663, XP009158777, ISSN: 0024-4201, DOI: 10.1007/BF02532171
- TZONG-HUEI LEE ET AL: "Heptadecanols from the leaves of Persea americana var. americana", FOOD CHEMISTRY, vol. 132, no. 2, 19 novembre 2011 (2011-11-19), pages 921-924, XP055025945, ISSN: 0308-8146, DOI: 10.1016/j.foodchem.2011.11.067
- Mostert, Mathilda Elizabeth: "Characterization of micro-components of avocado oil extracted with supercritical carbon dioxide and their effect on its oxidative stability", , 15 avril 2008 (2008-04-15), XP002675113, Extrait de l'Internet: URL:http://upetd.up.ac.za/thesis/available /etd-06062008-132406/ [extrait le 2012-05-02]
- BIZIMANA V: "Extraction, characterization, and prediction of the oxidative stability of avocado oil", DISSERTATION, 1997, pages 1-241, XP009158924, University of Minnesota

## Description

La présente description divulgue l'utilisation d'avocats mous entiers pour obtenir une huile d'avocat riche en insaponifiable. Avantageusement, ledit insaponifiable contient des acétogénines aliphatiques et/ou leurs dérivés. En particulier, ladite huile présente un faible indice d'acide, typiquement inférieur ou égal à 3 mgKOH/g. L'invention concerne un procédé d'obtention d'une huile d'avocat riche en insaponifiable à partir d'avocats mous entiers, ledit insaponifiable contenant des acétogénines aliphatiques et/ou leurs dérivés. L'invention concerne également une huile d'avocat riche en insaponifiable susceptible d'être obtenue par ledit procédé. La description divulgue également l'utilisation de l'huile d'avocat pour préparer un concentrat d'huile d'avocat enrichi en insaponifiable ou un insaponifiable d'avocat riche en furanes aliphatiques. Enfin, l'invention concerne un insaponifiable d'avocat riche en furanes aliphatiques ou un concentrat d'huile d'avocat enrichi en insaponifiable, ledit insaponifiable ou ledit concentrat étant susceptible d'être obtenu à partir de ladite huile d'avocat, pour son utilisation comme médicament, avantageusement dans la prévention et/ou le traitement des troubles du tissu conjonctif tels que l'arthrose, des pathologies articulaires telles que les rhumatismes, ou encore des maladies parodontales, telles que la gingivite ou la parodontite.

De par l'augmentation de ses surfaces cultivées, son accessibilité tout au long de l'année depuis le monde entier, l'augmentation du PIB par habitant, son intérêt alimentaire et ses valeurs nutritionnelles, la consommation de l'avocat tend à se généraliser dans le monde. Le marché de l'avocat est ainsi majoritairement axé sur la vente du fruit pour une utilisation dans l'alimentation soit du fruit directement, soit de ses produits dérivés, tels que le guacamole ou son huile.

La croissance du commerce mondial de l'avocat confirme l'intérêt grandissant pour ce fruit et sa valorisation, en particulier en raison de ses bénéfices nutritionnels *i.e.* pour la santé. Ainsi, ce marché mondial est basé sur une exportation massive des pays producteurs et une forte demande des pays importateurs entraînant un niveau de prix relativement élevé de cette matière première.

Cependant, la production industrielle de l'avocat et son commerce international doivent tenir compte des caractéristiques physiologiques particulières de ce fruit qui le rendent sensible au stockage et à la conservation.

Ainsi, la gestion des fruits après la récolte, celle-ci dépendant des fluctuations du marché de ce fruit de bouche, est un facteur primordial qui influe directement sur le rendement de production des agriculteurs (prélèvements et floraison) et la maitrise de leurs niveaux de pertes.

Lors de son développement et de sa maturation, l'avocat passe par plusieurs stades qui correspondent aux différentes phases physiologiques de son évolution sur l'arbre et lors de sa récolte jusqu'à sa conservation.

Ainsi, au cours de sa vie sur l'arbre, le fruit subit une phase de croissance, puis de maturation pour enfin être conduit vers la sénescence.

Durant la période de croissance du fruit, le végétal accumule des substances, telles que les protéines, les lipides ou l'amidon, les deux dernières étant assimilables à des substances dites de réserves carbonées. Ces substances servent à assurer la continuité du métabolisme après récolte. Ces réserves sont ensuite métabolisées en fonction des conditions de stockage et du temps, et cela plus ou moins rapidement.

La maturité physiologique du fruit correspond à une période de la vie durant laquelle s'effectuent des processus naturels qui complètent la croissance biométrique du fruit.

La maturation des fruits correspond à un ensemble de changements biochimiques et physiologiques conduisant à l'état de maturité et conférant au fruit ses caractéristiques chimiques et organoleptiques, comme l'arôme, la couleur, la dureté qui confère au fruit son caractère commercialisable et comestible.

Parmi les changements biochimiques subis par le fruit, certains sont visibles, tels que la dégradation des chlorophylles au cours de la maturation qui se résorbent en laissant apparaître progressivement d'autres pigments. La couleur du fruit provient de pigments localisés dans le cytoplasme ou dans les vacuoles cellulaires.

Un des changements importants lié à l'évolution du fruit est sa perte de fermeté. Cette transformation est attribuée à la dégradation, entre autre, des substances pectiques, molécules de soutien de la cellulose. Elle se caractérise principalement par une décomposition progressive de la paroi cellulaire entraînant un relâchement des fibres cellulosiques et conduisant à une perte de rigidité et de la fermeté du fruit. Elle est la résultante d'actions enzymatiques spécifiques, notamment représentées par les cellulases et polygalacturonases. Cette modification de la structure interne du fruit facilite alors les échanges cellulaires entre les différents organes du fruit et provoque aussi la libération et la diffusion de l'huile. Au-delà de l'état de maturité, le fruit entre dans une phase de sénescence conduisant à une modification biologique et physiologique, suivie d'une désorganisation cellulaire i.e. catabolisme. Lors de ces différentes phases, la composition de l'avocat se modifie. En particulier, des variations de ses teneurs en eau, en huile, en insaponifiable et en acides gras libres sont observées.

Lorsque ces fruits deviennent disponibles sur le marché, ils ne présentent souvent plus de degré de fermeté alors que leur processus de ramollissement a déjà débuté. Le délai de commercialisation des fruits est, alors, limité dans le temps.

L'extraction de l'huile à partir de ce fruit riche en lipides est ainsi rapidement apparue comme une utilisation

possible de ce végétal, notamment pour la valorisation de stocks commerciaux non exploitables sur le marché alimentaire, que l'on peut qualifier de sous-produits de la filière de l'avocat.

La mise en œuvre de tels fruits pour la production d'huile d'avocat limite le choix des procédés et des technologies à mettre en œuvre. Ainsi, la qualité et le stade de maturité sont des facteurs importants pour la préparation d'une huile d'avocat et vont conditionner le choix du procédé qui sera utilisé pour produire cette huile, la qualité et la composition de l'huile produite, notamment au niveau de son acidité, de la teneur et la composition de son insaponifiable.

Différentes méthodes d'extraction de l'huile à partir des fruits ont ainsi été développées parmi lesquelles on peut citer les techniques d'extraction par centrifugation, par pression ou par extraction à l'aide de solvants. La qualité des fruits utilisés, leur préparation, les technologies mises en œuvre, les conditions de trituration et de celles de conservation impactent grandement la qualité de l'huile produite, sa composition, son rendement d'extraction, ses propriétés et, par voie de conséquence, son utilisation.

Le procédé de production d'huile d'avocat principalement utilisé aujourd'hui utilise la technologie d'extraction par centrifugation. Les avocats, débarrassés de leur peau et de leur noyau, sont alors broyés et malaxés sous addition d'eau. Deux étapes de séparation par centrifugation sont ensuite nécessaires pour isoler l'huile. La qualité de l'huile obtenue par ce procédé dépend principalement de la qualité des fruits mis en œuvre, essentiellement liés à leur niveau de maturité et de ramollissement se rapprochant d'ailleurs de la sénescence : cet état entraîne un indice d'acide élevé du fait de l'utilisation de quantité importante d'eau et de la mise en activité des lipases natives du fruit dû à la décompartimentation cellulaire et à la présence d'eau libre et/ou de son émulsion avec l'huile en présence.

Par ailleurs, ce procédé qui apparaît très intéressant pour préparer une huile d'avocat destinée à des applications alimentaires ou cosmétiques présente un défaut important pour d'autres applications : un tel procédé est sélectif et ne permet pas d'extraire tout le potentiel en insaponifiable présent dans le fruit. En particulier, les composés polaires de l'insaponifiable, tels que les acétogénines aliphatiques du type alcools gras polyhydroxylés ou persins qui sont des précurseurs des furanes aliphatiques, ne sont pas extractibles du fruit par ce procédé. En effet, les liaisons relativement stables par lesquelles ils sont fixés au sein même de la matrice du fruit ne peuvent pas être rompues par ce type de procédé.

Des procédés basés sur la pression de la pulpe fraîche avec l'aide de produits tiers (silice végétale...) ou de vapeur ont également été développés, mais ils n'aboutissent eux aussi pas à une qualité d'huile riche en insaponifiable.

Des procédés intégrant un séchage des fruits à basse température (lyophilisation) avant extraction ont également été utilisés. Cependant, ces procédés, même s'ils incluent une étape d'extraction, par exemple par pression ou extraction par solvant, ne conduisent pas à la préparation d'une huile riche en acétogénines aliphatiques du type persins et conduisent donc à une huile à teneur relativement faible en insaponifiable et majoritairement constituée de stérols.

Plus généralement, des procédés à partir d'avocats frais ou préalablement déshydratés ont été développés qui intègrent l'utilisation de solvants de polarités spécifiques. Ces procédés visent à préparer des huiles d'avocat ou à extraire des constituants actifs de l'avocat pour des applications cosmétiques, alimentaires, nutraceutique ou pharmaceutiques. Cependant, au regard de leur développement industriel, de par la toxicité et la stabilité chimique du ou des solvants mis en œuvre, ainsi que d'un point de vue économique et environnemental, la viabilité de ces procédés reste contestable.

Le document BIZIMANA V (1997) divulgue des méthodes d'extraction d'huile par solvant, tel que l'hexane, l'éthanol ou l'acétone. En particulier, BIZIMANA compare deux méthodes d'extraction par solvant (chloroforme/méthanol versus acétone) des tissus d'avocat.

Par ailleurs, les procédés de préparation d'huile d'avocat par séchage à plus haute température et par pression des avocats déshydratés ne sont pas directement applicables aux avocats mous du fait des propriétés mécaniques de cette qualité de fruits. En effet, les fruits mous ne se prêtent pas aux conditions habituelles des opérations de tranchage et de séchage usuellement utilisées. Pour l'homme de l'art, il n'était aucunement probable, ni évident que des produits biologiques mous et pâteux, humides, semi-séchés ou séchés, puissent être traités par la technologie d'extraction des huiles par pression.

D'autre part, les exigences économiques liées au prix de revient élevé de l'huile d'avocat obligent à acquérir une parfaite maîtrise des rendements de pression et à obtenir une fiabilité technique du procédé industriel mis en œuvre.

Ainsi, il existait un besoin de mettre au point un nouveau procédé visant à obtenir une huile de qualité à partir de fruits mous qui constituent des sous-produits de l'avocat de bouche, en particulier avec un bon rendement tout en valorisant leur potentiel en insaponifiable.

De plus, les exigences des procédés développés pour extraire l'insaponifiable à partir des huiles d'avocats, notamment ceux intégrant une étape de concentration de la fraction insaponifiable par distillation moléculaire, imposent la mise œuvre d'une huile présentant un indice d'acide faible.

La présente invention vient combler ce besoin. La Demanderesse a ainsi découvert un nouveau procédé d'obtention d'une huile de qualité à partir des avocats mous avec un rendement satisfaisant tout en conduisant à l'obtention d'une teneur en insaponifiable élevée, en extrayant les composés polaires tels que les acétogénines aliphatiques, en limitant l'action des enzymes de type lipases, en particulier en limitant, voire annulant, l'hydrolyse des glycérides

tels que les triglycérides de l'huile, et présentant ainsi un indice d'acide faible. Le procédé selon la présente invention permet ainsi d'obtenir une huile présentant une teneur élevée en acétogénines aliphatiques et leurs dérivés.

Par ailleurs, le procédé selon la présente invention permet d'obtenir, avec un rendement élevé, une huile d'avocat riche en insaponifiable, qui peut avantageusement être incorporée au sein de compositions cosmétiques, dermatologiques, pharmaceutiques ou de dispositifs médicaux, ou encore dans des compositions alimentaires, des compléments alimentaires ou des nutraceutiques, à visée humaine ou animale.

En outre, à partir de l'huile selon la présente invention, on peut avantageusement extraire un insaponifiable d'avocat riche en furanes aliphatiques qui peut, lui-même, être incorporé au sein de compositions cosmétiques, dermatologiques, pharmaceutiques ou de dispositifs médicaux, ou encore dans des compositions alimentaires, des compléments alimentaires ou des nutraceutiques, à visée humaine ou animale.

La présente description divulgue ainsi l'utilisation d'avocats mous entiers pour obtenir une huile d'avocat riche en insaponifiable, ledit insaponifiable contenant des acétogénines aliphatiques et/ou leurs dérivés, dans laquelle la teneur en insaponifiable de l'huile est d'au moins 3% en masse, par rapport à la masse totale de l'huile, et la teneur en acétogénines aliphatiques et/ou leurs dérivés de l'huile est d'au moins 2% en masse, par rapport à la masse totale de l'huile.

En particulier, la présente description divulgue l'utilisation d'avocats mous entiers pour obtenir par pression mécanique une huile d'avocat contenant au moins 3% en masse d'insaponifiable par rapport à la masse totale de l'huile, ledit insaponifiable contenant des acétogénines aliphatiques et/ou leurs dérivés, dans laquelle la teneur en acétogénines aliphatiques et/ou leurs dérivés de l'huile est d'au moins 2% m/m, les avocats mous ayant une force de résistance à la pénétration dans la chair inférieure ou égale à 3 $kg/cm^2$, ladite force de résistance à la pénétration étant mesurée à l'aide d'un pénétromètre.

La plupart des variétés d'avocat peuvent être utilisées dans le cadre de la présente invention pour produire de l'huile avec les caractéristiques recherchées, dans la mesure où elles renferment le potentiel qualitatif et quantitatif en composés spécifiques.

De manière particulièrement avantageuse, le procédé selon l'invention est appliqué aux variétés les plus cultivées et représentant la *quasi* totalité des tonnages exportés et commercialisés au niveau mondial, préférentiellement les variétés *hass* et *fuerte.*

Par le terme d'avocats « entiers », on entend, au sens de la présente invention, des avocats contenant la peau, la pulpe et le noyau distribués dans leur intégrité.

Typiquement, les avocats mous selon l'invention ont un degré de ramollissement équivalent à celui d'une consommation immédiate de l'avocat, et excluent la possibilité d'un prétraitement mécanique par tranchage.

Avantageusement selon la présente invention, les avocats mous sont caractérisés par la fermeté de leur chair mesurée à l'aide d'un pénétromètre et définie par une force de résistance à la pénétration.

Selon la présente invention, les avocats mous ont une force de résistance à la pénétration dans la chair inférieure ou égale à 3 $kg/cm^2$, typiquement inférieure ou égale à 2 $kg/cm^2$, par exemple inférieure ou égale à 1 $kg/cm^2$.

Typiquement selon la présente invention, la force de pénétration est mesurée à l'aide d'un pénétromètre du type PCE-PTR 200 ou FT 327, qui mesure la force en kilogramme nécessaire pour faire pénétrer un embout calibré dans le fruit. Avantageusement, le fruit est pelé avant de faire la mesure afin de s'affranchir de la résistance de la peau (tégument) et de la variabilité relative aux différentes variétés d'avocat testées. La tige, appelée pointal ou embout, utilisée pour cette mesure a un diamètre nominal de l'ordre de 6 à 11,3 mm.

L'huile obtenue selon la présente invention est riche en insaponifiable. Par le terme de « riche en insaponifiable », on entend une huile qui contient au moins 3% en masse d'insaponifiable, avantageusement au moins 5% en masse d'insaponifiable, par rapport à la masse totale de l'huile. Par exemple, l'huile contient entre 3 et 12% en masse d'insaponifiable, en particulier 5 à 12%, plus particulièrement 8 à 12%, en masse d'insaponifiable, par rapport à la masse totale de l'huile.

L'insaponifiable comprend l'ensemble des constituants d'un corps gras qui après saponification en milieu fortement alcalin, sont très peu solubles ou insolubles dans l'eau, et solubles dans des solvants organiques, tels que l'éther éthylique, les hydrocarbures aromatiques, les solvants chlorés....

L'insaponifiable est donc composé de tous les constituants non hydrolysables du corps gras, ainsi que ceux résultant majoritairement de la saponification d'esters non glycéridiques d'acides gras (esters de stérols, cires, esters de tocophérols...).

Quatre grands groupes ou familles de substances sont généralement présents dans la plupart des insaponifiables d'huiles végétales. Le groupe le plus important en masse est représenté par celui qui rassemble les stérols incluant les alcools triterpéniques pentacycliques et les 4-méthylstérols. Le second groupe est constitué par les tocophérols pouvant intégrer des tocotriènols. Les deux autres groupes sont les alcools aliphatiques et les hydrocarbures aliphatiques saturés et insaturés.

La composition de l'insaponifiable d'avocat se différentie de la composition classiquement trouvée dans les huiles végétales car elle intègre majoritairement des constituants spécifiques de l'avocat. La fraction majoritaire de

l'insaponifiable d'avocat est représentée par le groupe des furanes aliphatiques. La deuxième famille de molécules regroupe les alcools gras polyhydroxylés. Le troisième groupe est constitué par les stérols incluant les alcools triterpéniques pentacycliques et les 4-méthylstérols. Les autres groupes sont minoritaires.

L'insaponifiable contenu dans l'huile selon l'invention contient des acétogénines aliphatiques et/ou leurs dérivés.

Dans le cadre de l'invention, les acétogénines aliphatiques et leurs dérivés sont des alcools gras polyhydroxylés et leurs dérivés acétylés, et/ou des 1,2-dihydroxy-4-oxo-aliphatiques alcools et leurs dérivés acétylés de type «persins», et/ou des furanes aliphatiques, ainsi que leurs mélanges.

Les alcools gras polyhydroxylés sont encore appelés Fraction I. Il s'agit notamment de triols du type 1,2,4-trihydroxy à longues chaînes acétyléniques et oléfiniques. Les dérivés acétylés des alcools gras polyhydroxylés sont typiquement en position 1, 2 ou 4.

Les 1,2-dihydroxy-4-oxo-aliphatiques alcools sont encore appelés persins, et sont des précurseurs de la Fraction H. Il s'agit notamment des diols cétones du type 1-2-dihydroxy-4-oxo à longues chaînes acétyléniques et oléfiniques. Les dérivés acétylés de ces composés sont typiquement en position 1.

Les persins se trouvent typiquement dans les idioblastes, cellules oléagineuses dans le cas de l'avocat.

Par exemple, on peut citer particulièrement les persins de structure moléculaire suivante :

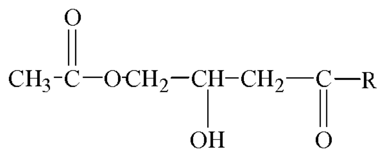

Les furanes aliphatiques sont encore appelés lipides furaniques, ou plus communément avocadofuranes ou Fraction H. Il s'agit notamment de dérivés des persins comportant un groupement furane, qui résultent en particulier de la transformation chimique par déshydratation et cyclisation intramoléculaire des persins extraites de l'avocat. A titre d'exemple, on peut citer les 2-alkyl furanes.

Avantageusement, l'insaponifiable de l'huile selon la présente invention est riche en acétogénines aliphatiques et/ou leurs dérivés. Selon la présente invention, l'huile contient au moins 2% en masse d'acétogénines aliphatiques et/ou de leurs dérivés, typiquement au moins 3% en masse d'acétogénines aliphatiques et/ou de leurs dérivés, par rapport à la masse totale de l'huile. Par exemple, l'huile contient entre 2 et 10%, en particulier entre 3 et 10%, plus particulièrement entre 5 et 8% en masse d'acétogénines aliphatiques et/ou de leurs dérivés, par rapport à la masse totale de l'huile.

Selon la présente invention, l'huile contient au moins 3% en masse d'insaponifiable, par rapport à la masse totale de l'huile, et contient au moins 2% en masse d'acétogénines aliphatiques et/ou de leurs dérivés, par rapport à la masse totale de l'huile.

Dans un autre mode de réalisation particulier selon la présente invention, l'huile contient au moins 5% en masse d'insaponifiable, par rapport à la masse totale de l'huile, et contient au moins 3% en masse d'acétogénines aliphatiques et/ou de leurs dérivés, par rapport à la masse totale de l'huile.

Typiquement, l'insaponifiable de l'huile selon l'invention contient des stérols. Par exemple, on peut trouver du β-sitostérol comme représentant majoritaire des 4-desméthysterols, du citrostadiènol pour la famille des 4-monométhylstérols, et/ou du 24-méthylènecycloarténol pour la famille des 4,4-diméthylstérols.

Selon une caractéristique particulière de l'invention, l'huile contient au moins 0,5 % en masse de stérols, avantageusement au moins 0,8% en masse de stérols, par rapport à la masse totale de l'huile.

Selon l'invention, les avocats mous entiers sont broyés, puis séchés à haute température entre 60 et 150°C, jusqu'à l'obtention d'une humidité résiduelle inférieure ou égale à 5%, avant l'obtention de l'huile par pression mécanique.

Selon la présente invention, suite au broyage et au séchage des avocats, 1 à 5 % d'eau ou de vapeur d'eau par rapport à la masse des avocats secs broyés, est additionné avant l'obtention de l'huile par pression mécanique.

Il a en effet été découvert que lorsque l'on intègre une étape d'injection d'eau ou de vapeur d'eau au sein des avocats séchés, ceci permet d'obtenir une huile d'avocat riche en insaponifiable avec un rendement élevé.

De manière particulièrement avantageuse, l'huile selon l'invention présente un faible indice d'acide, généralement inférieur ou égal à 5 mgKOH/g, avantageusement inférieur ou égal à 3 mgKOH/g, typiquement inférieur ou égal à 1 mgKOH/g.

La présente description divulgue un procédé d'obtention d'une huile d'avocat riche en insaponifiable à partir d'avocats mous entiers, dans laquelle ledit insaponifiable contient des acétogénines aliphatiques et/ou leurs dérivés, comprenant au moins les étapes successives suivantes :

- Broyage des avocats mous, conduisant avantageusement à l'obtention d'une granulométrie du broyat comprise entre 2 et 20 mm, en particulier entre 2 et 10 mm,

- Séchage du broyat à haute température, avantageusement à une température comprise entre 60 et 150°C, en particulier entre 65 et 120°C, par exemple entre 70 et 100°C, typiquement entre 80 et 100°C,
- Extraction de l'huile avantageusement par pression mécanique.

La présente invention a pour objet un procédé d'obtention d'une huile d'avocat à partir d'avocats mous entiers, ladite huile contenant au moins 3% en masse insaponifiable par rapport à la masse totale de l'huile, dans laquelle ledit insaponifiable contient des acétogénines aliphatiques et/ou leurs dérivés, les avocats mous ayant une force de résistance à la pénétration dans la chair inférieure ou égale à 3 kg/$cm^2$, ladite force de résistance à la pénétration étant mesurée à l'aide d'un pénétromètre, comprenant les étapes successives suivantes :

(1) Broyage des avocats mous, conduisant à l'obtention d'une granulométrie du broyat comprise entre 2 et 20 mm, en particulier entre 2 et 10 mm,
(2) Séchage du broyat à haute température, comprise entre 60 et 150°C, en particulier entre 65 et 120°C, par exemple entre 70 et 100°C, typiquement entre 80 et 100°C, jusqu'à l'obtention d'une humidité résiduelle inférieure ou égale à 5%,
(3) Addition d'eau aux avocats séchés par ajout de 1 à 5% d'eau ou de vapeur d'eau par rapport à la masse des avocats secs broyés, puis avantageusement homogénéisation par malaxage, avant introduction dans la presse, puis
(4) Extraction de l'huile par pression mécanique.

Il a été découvert que les fruits mous ne pouvaient pas se prêter aux conditions usuelles des opérations de tranchage ou de séchage opérées sur des avocats durs.

En effet, de par la constitution en trois parties distinctes du fruit, la peau, la pulpe et le noyau, son comportement lors du tranchage va être très variable en fonction de son degré de maturité et de fermeté de la pulpe et de la peau. Dans la première phase suivant sa récolte, le fruit possède une homogénéité de structure et de dureté entre les trois parties, favorable à son tranchage. Dès lors que la pulpe commence à se ramollir, la dureté compartimentaire du fruit (pulpe, peau, noyau) devient très hétérogène et empêche tout tranchage industriel du fait de la présence du noyau qui reste très dur et de la perte de consistance de la peau et de la pulpe.

Par ailleurs, il a été découvert que le séchage des fruits mous représentait un obstacle majeur à la mise en œuvre des procédés connus de l'homme de l'art afin d'extraire de l'huile d'avocat riche en sa fraction insaponifiable.

Le séchage des fruits mous entiers sans préparation ne donne pas de résultats satisfaisants, car il aboutit à un séchage hétérogène en eau libre résiduelle favorable à l'apparition de réactions parasites et hétérogènes, facteurs de dégradation de l'huile et de son insaponifiable.

Le broyage et le séchage du fruit mou dans des conditions non maîtrisées aboutit aux mêmes phénomènes et à la production d'une huile de qualité très variable limitant ou pénalisant ses utilisations.

La présente invention vient ainsi proposer une solution pour la préparation, le conditionnement éventuel, et le séchage des avocats mous, garantissant la préparation d'une huile d'avocat de qualité. Cette huile est notamment caractérisée par un indice d'acide faible, une teneur en insaponifiable élevée et une composition particulière de cet insaponifiable.

Dans le cadre de la présente invention, les avocats mous subissent une première étape de broyage (1). Cette étape permet de fractionner de façon efficace les différentes parties de l'avocat mou.

Dans un mode de réalisation particulier selon la présente invention, le broyage (1) est réalisé sur les avocats entiers constitués de la peau, de la pulpe et du noyau. Le broyage (1) permet avantageusement de déchiqueter la peau, de morceler le noyau et de malaxer le mélange afin d'obtenir une dispersion et une granulométrie homogènes du broyat (particules et morceaux obtenus) dans la pulpe d'avocat.

Typiquement, les broyeurs utilisés s'adaptent à la très grande différence de taille, de texture et de dureté des différentes parties qui composent l'avocat : peau, pulpe et noyau. Ainsi, la technologie de broyeurs à mettre en œuvre doit permettre de traiter des matériaux présentant des parties très dures (noyau) et des parties plus tendres (peau), voire très molles (pulpe).

Le broyage (1) est avantageusement réalisé à l'aide d'un broyeur de type à couteaux ou à rouleaux dentelés.

La configuration et le réglage doivent cependant être généralement adaptés en fonction de la taille des fruits, de leur maturité et de leur qualité (biométrie noyau, pulpe et peau) pour aboutir à la granulométrie avantageusement recherchée.

Dans un autre mode de réalisation particulier selon la présente invention, le broyage (1) est précédé par une étape préalable de séparation des différentes parties du fruit mou avant broyage. Des technologies efficaces existent pour séparer les noyaux et la peau de la pulpe de l'avocat. Ces différentes parties peuvent alors être traitées indépendamment par broyage sur des équipements adaptés avant d'être re-mélangées puis séchées.

Typiquement, les avocats mous sont traités tout d'abord sur une machine de type épureuse qui permet d'obtenir d'un côté la pulpe et de l'autre côté les noyaux et la peau.

Généralement, le mélange peau et noyau est ensuite broyé dans un broyeur du type broyeur à couteaux ou tous autres broyeurs appropriés pour obtenir une granulométrie en conformité avec les spécifications avantageusement attendues et décrites notamment ci-dessous.
Le broyat est ensuite re-mélangé à la pulpe dans un malaxeur et dans les proportions appropriées pour obtenir un mélange homogène présentant une dispersion régulière des particules de peau et de noyau dans la pulpe.
Ainsi, typiquement dans ce mode de réalisation, on procède à la succession des étapes suivantes :

- Séparation du noyau et de la peau de la pulpe des avocats,
- Broyage des différentes parties isolées des avocats mous, puis
- Malaxage des différentes parties broyées afin d'obtenir une dispersion homogène des particules de peau et de noyau dans la pulpe.

De manière particulièrement avantageuse selon l'invention, le broyage (1) est réalisé de telle manière à obtenir une taille spécifique des particules et morceaux, répondant à une fourchette de granulométrie qui donne au broyat une texture adaptée à un séchage rapide. Cette texture se caractérise par un aspect visuel non continu, les morceaux étant détectables généralement dans la masse sans utilisation d'un instrument optique complémentaire. Typiquement, la surface du broyat n'est pas lisse et comporte des aspérités représentées par les particules de noyau et de peau. Lors du séchage, le broyat ne forme pas une masse compacte, mais des blocs facilement friables.
De façon plus précise, la taille des particules est choisie entre 2 et 20 mm, et encore plus particulièrement entre 2 et 10 mm.
De manière avantageuse selon l'invention, le mélange, une fois broyé, doit être réparti de façon adaptée pour garantir une homogénéité du séchage (2) qui suit avec la plus grande efficacité. Différentes techniques sont applicables, telles que le malaxage et un conditionnement éventuel particulier, tel que l'étalement en couche mince, le dépôt de la pâte en tas réguliers à l'aide de buses ou de peignes, la formation de volumes structurés à l'aide de filières ou par extrusion.
Le broyat homogénéisé est réparti avantageusement de façon judicieuse pour obtenir lors de l'étape suivante de séchage (2) une évaporation rapide de l'eau, un accroissement maîtrisé de la température du produit, un séchage homogène et une adhérence minimale au support de séchage.
Ainsi, typiquement, dans un mode de réalisation particulier, suite au broyage (1) et préalablement au séchage (2), on procède au conditionnement du broyat afin d'augmenter la surface du broyat à sécher, avantageusement par étalement en couche mince, typiquement pour conduire à un film de faible épaisseur avantageusement comprise entre 0,5 et 5 cm, en particulier entre 1 et 2 cm, ou encore par mise en forme permettant d'optimiser la surface d'évaporation du type extrusion ou passage dans une filière.
Cette opération de conditionnement peut ainsi faire appel à différentes techniques, telles que :

- Répartition sur des claies, plateaux perforés, grillage de séchage ou sur une bande transporteuse, en un film d'épaisseur régulière, par exemple par passage entre deux rouleaux ;
- Dépôt de « pâtés » par une machine doseuse ou passage dans une filière ;
- Extrusion ;
- Ou toutes autres machines adaptées pour produire une structure régulière propice à un séchage homogène et reproductible.

L'étape suivante de déshydratation ou séchage (2) contrôlé du broyat a pour but d'extraire l'eau du milieu et de rendre extractibles les composés polaires de l'insaponifiable. Elle est réalisée, en particulier, grâce à une technologie spécifique et à une température choisie pour optimiser les besoins en énergie et limiter les réactions indésirables. En effet, une température trop basse limiterait la vitesse d'évaporation de l'eau et favoriserait l'action des lipases, entraînant l'hydrolyse des glycérides et l'augmentation de l'indice d'acide du milieu. Une température trop élevée favoriserait le phénomène de croûtage, ainsi que la dégradation thermique ou oxydative ou non-oxydative (réaction de Maillard) des composés fragiles et réactifs de l'insaponifiable ou des composés insaturés de l'huile.
Il a ainsi été découvert que, dans le but d'obtenir une huile de composition souhaitée, il était recommandé d'utiliser une étape de séchage à température ménagée et contrôlée. La teneur en eau très élevée de l'avocat ($\approx$ 75%) requiert ainsi une technique de séchage très efficace et spécifique pour garantir une évaporation rapide n'induisant pas de dégradation des constituants propres du fruit.
En effet, le séchage des avocats mous, sans préparation particulière et opéré comme cela est fait usuellement dans l'art antérieur, ne conduit pas à l'obtention d'une huile d'avocat de qualité satisfaisante. La barrière de la peau et la texture de l'avocat rendent la migration de l'eau vers la surface très difficile et limitent son évaporation. Sous ces conditions de températures, le milieu fortement hydraté à l'intérieur de la pulpe peut rendre l'action des lipases très favorable conduisant à un indice d'acide élevé sur l'huile extraite, ainsi qu'à la dégradation même de son insaponifiable.
En outre, une séparation de la pulpe par élimination du noyau et de la peau, puis un séchage de la pulpe

malaxée en couche mince permet d'améliorer le séchage, mais ne conduit pas à une huile avec les qualités souhaitée. Il se forme une croûte en surface au cours du séchage qui bloque le processus d'évaporation et qui maintient une humidité résiduelle au cœur de la pâte favorable à une action significative des lipases. La variation de l'épaisseur de la couche lors du séchage n'a pas d'effet significatif sur la maîtrise de cette réaction parasite.

Or, de façon très surprenante, il a été découvert que le séchage de l'avocat, lorsqu'il est réalisé à partir d'un mélange broyé incluant la pulpe, le noyau et la peau, comme cela est fait dans la présente invention, conduit à une déshydratation rapide et optimum avec une activité des lipases très limitée.

L'utilisation du mélange des trois parties apporte également un avantage important lors du séchage, car il empêche l'adhérence de la matière sur les plateaux de séchage. En effet, le broyat obtenu à partir des avocats entiers se décolle très facilement des plateaux après séchage contrairement au broyat de la pulpe qui colle au métal et empêche un déchargement rapide.

Il a également été découvert de manière surprenante que la granulométrie obtenue lors du broyage (1) impactait de façon significative la cinétique du séchage (2) et la qualité de l'huile extraite.

La taille des particules obtenues suite au broyage doit conduire avantageusement à l'obtention d'un mélange hétérogène régulier qui évite la formation d'une masse compacte lors du séchage qui bloquerait la migration de l'eau vers la surface et limiterait la surface d'évaporation.

De manière avantageuse selon l'invention, la texture hétérogène régulière favorise la circulation de l'air à l'intérieur de la masse et améliore l'évaporation de l'eau. Elle évite ainsi la formation d'une surface lisse en contact avec la surface des claies de séchage, limitant ainsi l'adhérence entre les deux surfaces.

Selon l'invention, le séchage (2) du broyat est réalisé à température ménagée et contrôlée, comprise entre 60 et 150°C, en particulier entre 65 et 120°C, par exemple entre 70 et 100°C, typiquement entre 80 et 100°C.

Selon une caractéristique particulière de l'invention, le séchage (2) du broyat est réalisé pendant 8 à 78 h, avantageusement pendant 10 à 24 h.

Le séchage (2) selon l'invention peut être réalisé en particulier par séchage sous courant d'air chaud ou sous atmosphère contrôlée (ex. azote), par séchage à pression atmosphérique ou sous vide, ou par séchage par micro-ondes.

Dans le cadre du présent procédé, pour des raisons de facilité de mise en œuvre industrielle et pour des raisons de coût, le séchage en séchoirs ventilés, en couche mince et sous courant d'air chaud, à une température comprise entre 80 et 100°C, pendant 8 à 72 heures est préféré.

A l'issue du séchage (2), le broyat séché présente un taux d'humidité résiduelle inférieure ou égale à 5% m/m.

L'humidité résiduelle est typiquement mesurée à l'aide d'une méthode thermogravimétrique par séchage IR. D'autres méthodes peuvent également être mises en œuvre, telles que la méthode de perte au séchage en étuve ou la méthode de titrage Karl Fischer.

Au cours du séchage, trois phases successives sont typiquement observées :

- Une phase de montée en température du produit jusqu'à la température humide de l'air de séchage,
- Une phase de séchage à vitesse constante qui correspond à une évaporation en surface du fruit et à la migration de l'eau libre de l'intérieur du produit vers la périphérie,
- Une phase de séchage à vitesse décroissante pendant laquelle la disponibilité de l'eau à la surface diminue du fait de la limitation de sa vitesse de migration à l'intérieur du fruit. A ce stade du séchage, il ne reste plus d'eau libre, mais principalement de l'eau liée beaucoup moins disponible à l'évaporation. Il est cependant particulièrement avantageux d'abaisser sa teneur résiduelle au maximum pour stabiliser le végétal et ainsi bloquer les développements microbiens et limiter les transformations internes par des actions enzymatiques résiduelles. Pour l'avocat, une teneur résiduelle de 5% est typiquement considérée comme maximum.

Une humidité résiduelle inférieure ou égale à 5% joue aussi un rôle important dans la consistance des avocats séchés, leur conférant une texture solide cassante favorable pour résister aux contraintes physiques développées pendant la pression mécanique. Au-delà de 5% d'humidité, l'avocat séché présente une consistance molle qui conduit, lors de la pression, à la formation d'une purée sans consistance suffisante pour être pressée efficacement.

Il a été trouvé que, même avec une humidité résiduelle inférieure ou égale à 5 % et une consistance propice à la pression, ces conditions ne sont pas totalement suffisantes pour atteindre des conditions de pression permettant un rendement d'extraction de l'huile élevé.

Il a ainsi été découvert de façon surprenante qu'un réajustement de l'humidité résiduelle (3) par addition de 1 à 5% d'eau ou de vapeur d'eau, par exemple de 1 à 3 % d'eau ou de vapeur d'eau, par rapport à la masse des avocats secs, permettait d'accroitre considérablement le taux d'extraction de l'huile, et de ce fait l'efficacité de la pression.

Cette opération de réajustement/addition d'eau (3) doit être réalisée juste avant l'introduction des fruits séchés dans la presse par ajout d'eau purifiée ou de vapeur d'eau pour que les avocats secs se saturent en humidité en surface sans perdre leur consistance ferme et craquante afin qu'aucun ramollissement n'apparaisse.

Ce taux d'humidité résiduelle pour être efficace ne peut être obtenu que par déshydratation préalable (2) des

avocats, puis réajustement par addition d'eau (3). En effet, une déshydratation partielle contrôlée directe n'aboutit pas à une texture de produit compatible avec la pression, comme il a été signalé précédemment.

De façon particulière selon l'invention, l'addition d'eau (3) dans les avocats séchés est réalisée par ajout contrôlé d'eau ou de vapeur d'eau sur les avocats séchés et broyés, puis homogénéisation par malaxage typiquement dans un mélangeur planétaire, avantageusement pendant 1/2h à 1h.

De façon plus particulière selon l'invention, l'addition d'eau (3) dans les avocats séchés est réalisée en continu dans un convoyeur du type vis sans fin. L'eau ou la vapeur d'eau est ajoutée sur les avocats en tête de convoyeur et l'homogénéisation est obtenue par agitation dans le convoyeur, lors du déplacement du produit. Le dimensionnement du convoyeur doit permettre de garantir typiquement un temps de séjour minimum de 1/2h des avocats séchés.

De façon encore plus particulière selon l'invention, le convoyeur est utilisé pour alimenter la presse d'extraction.

L'étape d'extraction (4) de l'huile est ainsi tout particulièrement avantageusement précédée d'une étape d'addition d'eau (3) dans les avocats séchés par ajout de 1 à 5%, de préférence de 1 à 3%, d'eau ou de vapeur d'eau par rapport à la masse des avocats séchés.

L'étape d'extraction (4) de l'huile est mise en œuvre par une pression mécanique de la matière sèche après addition d'eau. Usuellement, pour pouvoir travailler avec efficacité, les presses d'extraction doivent recevoir une matière contenant une teneur en fibres et matière organique adaptée qui confère au tourteau produit une consistance qui permet d'atteindre en tête de presse une pression élevée indispensable pour garantir un rendement de pression adapté.

L'étape d'extraction (4) selon l'invention est généralement complétée par une filtration qui élimine les particules solides et garantit la limpidité de l'huile produite.

Le procédé selon la présente invention permet de produire ainsi une huile d'avocat de qualité avec une composition particulière, notamment possédant un indice d'acide faible et un potentiel élevé en insaponifiable et une composition de cet insaponifiable particulière.

La présente invention a ainsi également pour objet une huile d'avocat riche en insaponifiable susceptible d'être obtenue par le procédé selon l'invention.

L'huile contient au moins 3% en masse d'insaponifiable, typiquement au moins 5% en masse d'insaponifiable, par rapport à la masse totale de l'huile. Par exemple, l'huile contient entre 3 et 12% en masse d'insaponifiable, en particulier 5 à 12%, plus particulièrement 8 à 12%, en masse d'insaponifiable, par rapport à la masse totale de l'huile.

L'huile contient au moins 2% en masse d'acétogénines aliphatiques et/ou de leurs dérivés, par rapport à la masse totale de l'huile, lesdits acétogénines aliphatiques et leurs dérivés étant tels que définis ci-dessus et étant des alcools gras polyhydroxylés et leurs dérivés acétylés, et/ou des 1,2-dihydroxy-4-oxo-aliphatiques alcools et leurs dérivés acétylés de type «persins», et/ou des furanes aliphatiques.

De manière particulièrement avantageuse, l'huile contient typiquement au moins 3% en masse d'acétogénines aliphatiques et/ou de leurs dérivés, par rapport à la masse totale de l'huile. Par exemple, l'huile contient entre 2 et 10%, en particulier entre 3 et 10%, plus particulièrement entre 5 et 8% en masse d'acétogénines aliphatiques et/ou de leurs dérivés, par rapport à la masse totale de l'huile.

Selon la présente invention, l'huile contient au moins 3% en masse d'insaponifiable, par rapport à la masse totale de l'huile, et contient au moins 2% en masse d'acétogénines aliphatiques et/ou de leurs dérivés, par rapport à la masse totale de l'huile.

Dans un autre mode de réalisation particulier selon la présente invention, l'huile contient au moins 5% en masse d'insaponifiable, par rapport à la masse totale de l'huile, et contient au moins 3% en masse d'acétogénines aliphatiques et/ou de leurs dérivés, par rapport à la masse totale de l'huile.

Typiquement, l'insaponifiable de l'huile selon l'invention contient des stérols.

Selon une caractéristique particulière de l'invention, l'huile contient au moins 0,5 % en masse de stérols, avantageusement au moins 0,8% en masse de stérols, par rapport à la masse totale de l'huile.

De manière particulièrement avantageuse, l'huile selon l'invention présente un faible indice d'acide, généralement inférieur ou égal à 5 mgKOH/g, avantageusement inférieur ou égal à 3 mgKOH/g, typiquement inférieur ou égal à 1 mgKOH/g.

La présente description divulgue également une composition contenant une huile d'avocat riche en insaponifiable, avantageusement à une concentration comprise entre 0,1 et 99,9% en masse, encore plus avantageusement de 30 à 70% en masse, par rapport à la masse totale de la composition.

La présente description divulgue également l'utilisation de l'huile d'avocat telle que définie ci-dessus pour préparer un concentrat d'huile d'avocat enrichi en fraction insaponifiable.

Par le terme de concentrat d'huile d'avocat enrichi en fraction insaponifiable, on entend au sens de la présente invention un concentrat d'huile d'avocat contenant une teneur élevée en insaponifiable, typiquement contenant au moins 20%, par exemple entre 30 et 60% d'insaponifiable en masse, par rapport à la masse totale du concentrat.

Avantageusement, l'insaponifiable du concentrat d'huile d'avocat selon l'invention contient des acétogénines aliphatiques et/ou leurs dérivés.

La présente description divulgue également l'utilisation de l'huile d'avocat telle que définie ci-dessus ou du

concentrat d'huile d'avocat tel que mentionné ci-dessus pour préparer un insaponifiable d'avocat riche en furanes aliphatiques.

Les étapes de concentration de l'huile en sa fraction insaponifiable pour préparer un concentrat tel que mentionné ci-dessus, et de préparation d'un insaponifiable d'avocat riche en furanes aliphatiques (lipides furaniques) à partir de l'huile ou du concentrat sont en particulier celles décrites ci-dessous.

La préparation du concentrat est généralement opérée par cristallisation à froid ou par distillation moléculaire. Avantageusement, le concentrat d'huile est préparé par distillation moléculaire, typiquement à une température comprise entre 180 et 260°C en maintenant une pression comprise entre $10^{-2}$ et $10^{-3}$ mmHg.

Cette étape de distillation moléculaire de l'huile d'avocat est de préférence réalisée en utilisant un dispositif choisi parmi les distillateurs moléculaires de type centrifuge et les dispositifs de type à film raclé.

La préparation de l'insaponifiable d'avocat riche en furanes aliphatiques à partir de l'huile ou du concentrat inclut généralement un traitement thermique de l'huile ou du concentrat à une température comprise entre 80 et 150°C, suivie d'une étape de saponification et d'extraction de l'insaponifiable, par exemple à l'aide d'un solvant.

Suite à l'extraction de l'insaponifiable, on peut procéder à des étapes complémentaires de purification ou de fractionnement.

La présente invention a également pour objet le concentrat d'huile d'avocat enrichi en fraction insaponifiable préparé à partir de l'huile d'avocat selon l'invention.

La présente invention a également pour objet l'insaponifiable total ou la fraction insaponifiable préparé(e) à partir de l'huile d'avocat selon l'invention ou du concentrat d'huile d'avocat selon l'invention.

La présente description divulgue également une composition contenant un insaponifiable préparé typiquement à partir de l'huile d'avocat selon l'invention ou du concentrat tel que mentionné ci-dessus, typiquement un insaponifiable total ou une fraction insaponifiable, avantageusement à une concentration comprise entre 0,1 et 99,9% en masse, encore plus avantageusement de 30 à 70% en masse, par rapport à la masse totale de la composition.

L'insaponifiable total renferme toutes les familles de constituants typiquement présents dans l'insaponifiable de l'huile d'origine considérée.

Une fraction insaponifiable résulte typiquement d'une ou plusieurs opérations de fractionnement, permettant d'obtenir avantageusement sous forme de fraction purifiée, un composé ou une famille de composés constituant de l'insaponifiable.

Avantageusement, la composition contient une huile d'avocat riche en insaponifiable selon la présente invention et/ou un concentrat préparé typiquement à partir de l'huile selon l'invention et/ou un insaponifiable préparé typiquement à partir de l'huile d'avocat ou du concentrat selon l'invention, typiquement un insaponifiable total ou une fraction insaponifiable.

La composition peut, en outre, comprendre d'autres actifs.

Parmi les actifs recommandés en association avec l'huile et/ou le concentrat et/ou l'insaponifiable selon l'invention, on peut citer les extraits végétaux, en particulier :

- Les huiles ou beurres végétaux tels que les huiles de Soja et/ou l'huile de Colza, l'huile de Lupin, avantageusement l'huile de Lupin blanc doux, ou un mélange de ces huiles ou beurres ;
- l'oléodistillat ou les concentrats d'huile végétale ou animale, notamment de tournesol, plus avantageusement des concentrats de Tournesol linoléiques, tels que l'huile de tournesol concentrée en insaponifiable (Soline®) commercialisée par les Laboratoires Expanscience, les huiles concentrées en insaponifiable du type huile de soja, de colza, de maïs ou de palme ;
- les insaponifiables de végétaux ou d'huile végétale, avantageusement des furanes d'avocat (Avocadofurane®), les insaponifiables d'Avocat et/ou de Soja, plus particulièrement un mélange d'insaponifiables d'Avocat furaniques et d'insaponifiables de Soja, avantageusement dans un rapport respectif d'environ 1/3-2/3 (tel que Piasclédine®300), les insaponifiables de soja, les insaponifiables stéroliques (typiquement des insaponifiables dont la teneur en stérols, en méthylstérols et en alcools triterpèniques est comprise entre 20 et 95 % en masse, de préférence 45-65 % en masse, par rapport à la masse totale de l'insaponifiable), les phytostérols, les esters de stérols et les dérivés vitaminiques.

En particulier, la composition contient un insaponifiable d'avocat selon l'invention en association avec un insaponifiable de soja, avantageusement dans un rapport d'environ 2/3 pour le soja et 1/3 pour l'avocat (tel que Piasclédine®300).

Enfin, la présente invention a également pour objet l'huile d'avocat riche en insaponifiable selon la présente invention telle que décrite ci-dessus ou le concentrat d'avocat tel que décrit ci-dessus ou l'insaponifiable tel que décrit ci-dessus ou la composition telle que décrite ci-dessus, pour son utilisation comme médicament, comme dispositif médical, comme agent dermatologique, comme agent cosmétique, ou comme nutraceutique, à visée humaine ou animale, avantageusement dans la prévention et/ou le traitement des troubles du tissu conjonctif tels que l'arthrose, des

pathologies articulaires telles que les rhumatismes, des maladies parodontales, telles que la gingivite ou la parodontite, ou encore dans la prévention et/ou le traitement des troubles du derme et/ou de l'hypoderme tels que le vieillissement cutané, les vergetures et la cellulite, ou encore des troubles de la barrière épidermique tels que les inflammations cutanées, l'eczéma atopique et les dermatites irritatives et/ou inflammatoires.

En particulier, la présente invention a pour objet un insaponifiable d'avocat riche en furanes aliphatiques ou un concentrat d'huile d'avocat enrichi en insaponifiable, ledit insaponifiable ou ledit concentrat étant susceptible d'être obtenu à partir de l'huile d'avocat selon l'invention, pour son utilisation comme médicament, dans la prévention et/ou le traitement des troubles du tissu conjonctif tels que l'arthrose, des pathologies articulaires telles que les rhumatismes, ou encore des maladies parodontales, telles que la gingivite ou la parodontite.

Par ailleurs, on entend par dispositif médical selon l'invention tout instrument, appareil, équipement, matière, produit, à l'exception des produits d'origine humaine, ou autre article seul ou en association, destiné par le fabricant à être utilisé chez l'homme à des fins médicales et dont l'action principale voulue n'est pas obtenue par des moyens pharmacologiques ou immunologiques ni par métabolisme, mais dont la fonction peut être assistée par de tels moyens.

Avantageusement, les compositions selon l'invention sont adaptées à une administration orale, telles qu'une composition pharmaceutique ou un médicament, un complément alimentaire ou une composition nutraceutique.

Selon une variante, les compositions selon l'invention sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Les exemples suivants sont donnés pour illustrer l'invention :

<u>Exemple 1 : Extraction d'une huile de fruits d'avocat mous : Influence de la granulométrie lors du broyage (1) et de l'addition d'eau (3) avant pression (4) aux avocats préalablement séchés</u>

3 kg d'avocats mous d'origine kényane, de calibre 18, de variété *hass* et de dureté mesurée à l'aide d'un pénétromètre comprise entre 0,1 et 0,3 kg/cm$^2$, sont broyés dans un broyeur à couteaux RETSCH du type SM 100 équipé selon la granulométrie souhaitée de tamis de fond à ouverture de maille de 20 mm, 10 mm ou 1 mm.

Le broyat est déposé sur un plateau de 0,158 m$^2$, sur une épaisseur de 2 cm.

Le plateau est ensuite disposé dans une étuve ventilée et le séchage est réalisé sous une circulation d'air à 80 $\pm$ 5 °C pendant 72 heures.

Le broyat séché est récupéré et broyé pour homogénéisation sur le broyeur à couteaux RETSCH du type SM 100 équipé de tamis de fond à ouverture de maille de 20 mm.

L'humidité résiduelle est mesurée à l'aide d'une méthode thermogravimétrique par séchage IR.

Suivant les essais, le broyat est ensuite hydraté ou non par vaporisation d'une quantité d'eau purifiée adaptée et par homogénéisation dans un mélangeur planétaire, juste avant d'être introduit dans la presse pour extraction de l'huile.

L'huile d'avocat est ensuite extraite par pression mécanique sur une presse à vis de laboratoire du type Komet.

Les quantités d'huile et de tourteau sont déterminées par pesée pour chaque essai et le rendement d'extraction est calculé par la formule suivante :

Masse d'huile récupérée x100 / (Masse d'huile récupérée + masse de tourteau).

L'analyse physico-chimique et chromatographique des huiles permet de comparer les compositions des différentes huiles obtenues.

| N° essai | Maille tamis | HR après séchage | % eau ajoutée | Rendement d'extraction | Teneur en acétogénines | Teneur en insaponifiable | IA |
|---|---|---|---|---|---|---|---|
| | mm | % | % | % | m/m | m/m | mgKOH/g |
| 1 | 1 | 6,0 | 0 | 17,2 | 3,24 | 3,99 | 3,1 |
| 2 | 10 | 1,5 | 0 | 16,6 | 3,67 | 4,40 | 0,9 |
| 3 | 10 | 1,5 | 3,6 | 43,3 | 3,71 | 4,30 | 1,7 |
| 4 | 20 | 1,4 | 3,9 | 41,6 | 3,56 | 4,43 | 1,4 |

L'essai n°1 met en évidence qu'une taille de particule correspondant à un broyage avec un tamis de maille 1 mm ne permet pas d'obtenir après 72h de séchage une humidité résiduelle (HR) inférieure à 5%, compromettant ainsi

la qualité du stockage des avocats séchés et aboutissant à un rendement de pression très faible et à une huile plus faiblement dosée en acétogénines et insaponifiable. Il conduit également à un indice d'acide (IA) supérieur à 3 de l'huile d'avocat obtenue.

Les essais n°2 et 3 de pression mettent en évidence que l'addition d'eau aux fruits séchés avant pression permet d'atteindre un rendement de pression élevé dans le cas de l'essai n°3, contrairement à l'essai n°2 dans lequel aucune opération d'addition d'eau n'a été mise en œuvre.

Les essais 3 et 4, comparativement à l'essai 1, mettent en évidence le bénéfice d'une taille de particules comprise entre 10 et 20 mm sur la composition de l'huile, notamment sur sa teneur en acétogénines et insaponifiable.

Exemple 2 : Extraction d'une huile de fruits d'avocat mous incluant notamment une étape de broyage (1) séparée des différentes parties de l'avocat et influence de l'addition d'eau (3) avant pression (4) aux avocats séchés (2)

3 kg d'avocats mous d'origine Kényane, de calibre 18, de variété *hass* et de dureté mesurée à l'aide d'un pénétromètre, comprise entre 0,1 et 0,3 kg/cm$^2$, sont dénoyautés et dépulpés, puis les deux parties (pulpe d'une part et noyau + peau d'autre part) sont broyées dans un broyeur à couteaux RETSCH du type SM 100 équipé selon la granulométrie souhaitée de tamis de fond à ouverture de maille de 10 mm.

Une partie de la pulpe broyée est remélangée avec sa partie correspondante de noyau + peau broyés dans les proportions d'origine (essai n°7). Une autre partie de la pulpe est séchée seule (essai n°6).

Les différents broyats sont répartis sur des plateaux sur une épaisseur de 2 cm. Le plateau est ensuite disposé dans une étuve ventilée et le séchage est réalisé sous une circulation d'air à 80 ± 5 °C pendant 72 heures.

Les broyats séchés sont récupérés et broyés pour homogénéisation sur le broyeur à couteaux RETSCH du type SM 100 équipé de tamis de fond à ouverture de maille de 20 mm.

L'humidité résiduelle est mesurée à l'aide d'une méthode thermogravimétrique par séchage IR.

Suivant les essais, le broyat est ensuite hydraté ou non par vaporisation d'une quantité d'eau purifiée adaptée et par homogénéisation dans un mélangeur planétaire, juste avant d'être introduit dans la presse pour extraction de l'huile.

L'huile d'avocat est ensuite extraite par pression mécanique sur une presse à vis de laboratoire du type Komet.

Les quantités d'huile et de tourteau sont déterminées par pesée pour chaque essai et le rendement d'extraction est calculé par la formule suivante :

$$\text{Masse d'huile récupérée x100 / (Masse d'huile récupérée + masse de tourteau).}$$

L'analyse physico-chimique et chromatographique des huiles permet de comparer les compositions des différentes huiles obtenues.

| N° essai | Partie | Maille tamis | HR après séchage | % eau ajoutée | Rendement d'extraction | Teneur en acétogénines | Teneur en insaponifiable | IA |
|---|---|---|---|---|---|---|---|---|
| | | mm | % | % | % | m/m | m/m | mgKOH/g |
| 5 | Avocat entier | 10 | 1,5 | 3,6 | 43,3 | 3,71 | 4,30 | 1,7 |
| 6 | Pulpe seule | 10 | 5,7 | 0 | 58,2 | 2,89 | 3,99 | 3,3 |
| 7 | Pulpe, noyau et peau remélangés | 10 | 1,6 | 1,9 | 43,7 | 3,67 | 4,40 | 1,0 |

L'essai n°6 met en évidence qu'un broyage de la pulpe seule, même avec une maille de 10 mm, ne permet pas d'obtenir après 72h de séchage une humidité résiduelle (HR) inférieure à 5%, compromettant ainsi la qualité du stockage des avocats séchés et aboutissant à une huile plus faiblement dosée en acétogénines et en insaponifiable. Il conduit également à un indice d'acide (IA) supérieur à 3 de l'huile d'avocat obtenue.

Les essais n°5 et 7 comparativement mettent en évidence qu'un broyage séparé des différentes parties de l'avocat, puis homogénéisation du mélange des broyats avant séchage d'une part, et le broyage de l'avocat dans son intégralité d'autre part donnent des résultats similaires en termes de rendement de pression après addition d'eau dans les fruits séchés et en termes de composition de l'huile obtenue.

Des essais complémentaires utilisant des pourcentages d'eau croissants lors de l'addition en eau du mélange pulpe, peau et noyau séché permettent de mettre en évidence le rôle de cette étape clé du procédé pour obtenir un rendement d'extraction maximal.

| N° essai | Partie | Maille tamis | HR après séchage | % eau ajoutée | Rendement d'extraction |
|---|---|---|---|---|---|
| | | mm | % | % | % |
| 8 | Pulpe, noyau et peau remélangés | 10 | 1,6 | 0 | 9,1 |
| 9 | | | | 1,9 | 43,7 |
| 10 | | | | 4,4 | 42,2 |

Les essais n° 9 et 10 mettent en évidence que l'addition d'eau aux fruits séchés avant pression permet d'atteindre un rendement de pression élevé, contrairement à l'essai n°8 dans lequel aucune opération d'addition en eau n'a été mise en œuvre.

<u>Exemple 3</u> comparatif :

<u>Extraction d'une huile par centrifugation à partir de fruits d'avocat mous entiers</u>

100 kg d'avocats mous d'origine mexicaine, de variété *hass,* sont traités sur une raffineuse du type à tamis rotatif pour séparer la peau et les noyaux.
La pulpe est ensuite traitée suivant les opérations suivantes :

- De l'eau est ajoutée à la pulpe obtenue et le mélange est chauffé à 80°C,
- Le mélange est ensuite soumis à une centrifugation pour séparer la partie liquide constituée d'huile et d'eau de la partie solide,
- L'huile est ensuite séparée de la phase aqueuse par une nouvelle étape de centrifugation,
- L'huile obtenue subit une dernière phase de filtration avant d'être conditionnée.

L'analyse physico-chimique et chromatographique de cette huile a donné les résultats suivants :

- Indice d'acide : 2,7 mg KOH/g,
- Teneur en acétogénines aliphatiques et/ou leurs dérivés : 0,55 g/100 g,
- Teneur en stérols : 0,52 g/100 g,
- Teneur en insaponifiable : 1,19 g/100 g.

L'huile obtenue présente un indice d'acide satisfaisant, mais montre une teneur en acétogénines aliphatiques et/ou leurs dérivés très faible, qui démontre l'inefficacité du procédé par centrifugation à extraire les composés polaires de l'insaponifiable.

## Revendications

1. Procédé d'obtention d'une huile d'avocat à partir d'avocats mous entiers, ladite huile contenant au moins 3% en masse insaponifiable par rapport à la masse totale de l'huile, dans laquelle ledit insaponifiable contient des acétogénines aliphatiques et/ou leurs dérivés, les avocats mous ayant une force de résistance à la pénétration dans la chair inférieure ou égale à 3 kg/$cm^2$, ladite force de résistance à la pénétration étant mesurée à l'aide d'un pénétromètre, comprenant les étapes successives suivantes :

(1) Broyage des avocats mous conduisant à l'obtention d'une granulométrie du broyat comprise entre 2 et 20 mm,
(2) Séchage du broyat à haute température entre 60 et 150°C, jusqu'à l'obtention d'une humidité résiduelle inférieure ou égale à 5%,
(3) Addition d'eau aux avocats séchés par ajout de 1 à 5 % d'eau ou de vapeur d'eau par rapport à la masse des avocats secs broyés, puis
(4) Extraction de l'huile par pression mécanique.

**2.** Procédé d'obtention d'une huile d'avocat selon la revendication 1, **caractérisé en ce que** le broyage (1) est réalisé sur les avocats entiers constitués de la peau, de la pulpe et du noyau, par déchiquetage de la peau, morcellement du noyau et malaxage du mélange afin d'obtenir une dispersion homogène du broyat, avantageusement à l'aide d'un broyeur de type à couteaux ou à rouleaux dentelés.

**3.** Procédé d'obtention d'une huile d'avocat selon la revendication 1, **caractérisé en ce que** le broyage (1) est réalisé par:

- Séparation du noyau et de la peau de la pulpe des avocats,
- Broyage des différentes parties des avocats mous, puis
- Malaxage des différentes parties broyées afin d'obtenir une dispersion homogène.

**4.** Procédé d'obtention d'une huile d'avocat selon la revendication 1, **caractérisé en ce que** le broyage (1) des avocats mous conduit à l'obtention d'une granulométrie du broyat comprise entre 2 et 10 mm.

**5.** Procédé d'obtention d'une huile d'avocat selon la revendication 1, **caractérisé en ce que** le séchage (2) du broyat est réalisé à haute température entre 70 et 100°C.

**6.** Procédé d'obtention d'une huile d'avocat selon l'une quelconque des revendications 1 à 5, **caractérisé en ce que** suite au broyage (1) et préalablement au séchage (2), on procède au conditionnement du broyat afin d'augmenter la surface du broyat à sécher, par étalement en couche mince, pour conduire à un film d'épaisseur comprise entre 0,5 et 5 cm, ou encore par mise en forme permettant d'optimiser la surface d'évaporation du type extrusion ou passage dans une filière.

**7.** Procédé d'obtention d'une huile d'avocat selon l'une quelconque des revendications 1 à 6, **caractérisé en ce que** le séchage (2) du broyat est réalisé pendant 8 à 78 h.

**8.** Procédé d'obtention d'une huile d'avocat selon la revendication 7, **caractérisé en ce que** le séchage (2) du broyat est réalisé pendant 10 à 24 h.

**9.** Huile d'avocat susceptible d'être obtenue par le procédé selon l'une quelconque des revendications 1 à 8, **caractérisée en ce qu'**elle contient au moins 3% en masse d'insaponifiable, par rapport à la masse totale de l'huile et au moins 2% en masse d'acétogénines aliphatiques et/ou de leurs dérivés, par rapport à la masse totale de l'huile, lesdits acétogénines aliphatiques et leurs dérivés étant des alcools gras polyhydroxylés et leurs dérivés acétylés, et/ou des 1,2-dihydroxy-4-oxo- aliphatiques alcools et leurs dérivés acétylés de type «persins», et/ou des furanes aliphatiques.

**10.** Huile d'avocat selon la revendication 9, contenant au moins 5% en masse d'insaponifiable, par rapport à la masse totale de l'huile, et/ou contenant au moins 3% en masse d'acétogénines aliphatiques et/ou de leurs dérivés, par rapport à la masse totale de l'huile.

**11.** Huile d'avocat selon la revendication 9 ou 10, **caractérisée en ce qu'**elle présente un indice d'acide inférieur ou égal à 5 mgKOH/g.

**12.** Huile d'avocat selon la revendication 11, **caractérisée en ce qu'**elle présente un indice d'acide inférieur ou égal à 3 mgKOH/g.

**13.** Huile d'avocat selon la revendication 11, **caractérisée en ce qu'**elle présente un indice d'acide inférieur ou égal à 1 mgKOH/g.

**14.** Huile d'avocat telle que définie à l'une quelconque des revendications 9 à 13 contenant au moins 3% en masse d'insaponifiable, par rapport à la masse totale de l'huile, insaponifiable d'avocat riche en furanes aliphatiques ou concentrât d'huile d'avocat enrichi en insaponifiable, ledit insaponifiable ou ledit concentrât étant susceptible d'être obtenu à partir de l'huile d'avocat telle que définie à l'une quelconque des revendications 9 à 13, pour son utilisation comme médicament, dans la prévention et/ou le traitement des troubles du tissu conjonctif tels que l'arthrose, des pathologies articulaires telles que les rhumatismes, ou encore des maladies parodontales, telles que la gingivite ou la parodontite.

**Patentansprüche**

**1.** Verfahren zur Gewinnung eines Avocadoöls aus ganzen weichen Avocados, wobei das Öl mindestens 3 Ma% Unverseifbares in Bezug auf die Gesamtmasse des Öls enthält, wobei das Unverseifbare aliphatische Acetogenine und/oder deren Derivate enthält, wobei die weichen Avocados eine Widerstandskraft gegenüber dem Eindringen in das Fruchtfleisch von unter oder gleich 3 $kg/cm^2$ haben, wobei die Widerstandskraft gegenüber dem Eindringen mit Hilfe eines Penetrometers gemessen wird, umfassend die folgenden aufeinanderfolgenden Schritte:

(1) Zerkleinern der weichen Avocados, das zu einer Partikelgröße des Zerkleinerungsprodukts führt, die zwischen 2 und 20 mm liegt,
(2) Trocknen des Zerkleinerungsprodukts bei hoher Temperatur zwischen 60 und 150°C bis zum Erhalt einer Restfeuchte von unter oder gleich 5%,
(3) Hinzufügen von Wasser zu den getrockneten Avocados durch Hinzufügen von 1 bis 5% Wasser oder Wasserdampf in Bezug auf die Masse der zerkleinerten trockenen Avocados, dann
(4) Extraktion des Öls durch mechanisches Pressen.

**2.** Verfahren zur Gewinnung eines Avocadoöls nach Anspruch 1, **dadurch gekennzeichnet, dass** das Zerkleinern (1) auf den ganzen Avocados durchgeführt wird, die von der Haut, dem Fruchtfleisch und dem Samen gebildet sind, durch Zerreißen der Haut, Zerkleinern des Samens und Mischen des Gemischs, um eine homogene Dispersion des Zerkleinerungsprodukts zu erhalten, in vorteilhafter Weise mit einem Zerkleinerer vom Typ mit Messern oder gezahnten Walzen.

**3.** Verfahren zur Gewinnung eines Avocadoöls nach Anspruch 1, **dadurch gekennzeichnet, dass** das Zerkleinern (1) durchgeführt wird durch:

- Trennen des Samens und der Haut vom Fruchtfleisch der Avocados,
- Zerkleinern der verschiedenen Teile der weichen Avocados, dann
- Mischen der verschiedenen zerkleinerten Teile, um eine homogene Dispersion zu erhalten.

**4.** Verfahren zur Gewinnung eines Avocadoöls nach Anspruch 1, **dadurch gekennzeichnet, dass** das Zerkleinern (1) der weichen Avodacos zur Gewinnung einer Partikelgröße des Zerkleinerungsprodukts führt, die zwischen 2 und 10 mm liegt.

**5.** Verfahren zur Gewinnung eines Avocadoöls nach Anspruch 1, **dadurch gekennzeichnet, dass** das Trocknen (2) des Zerkleinerungsprodukts bei hoher Temperatur zwischen 70 und 100°C durchgeführt wird.

**6.** Verfahren zur Gewinnung eines Avocadoöls nach einem der Ansprüche 1 bis 5, **dadurch gekennzeichnet, dass** das Zerkleinerungsprodukt nach dem Zerkleinern (1) und vor dem Trocknen (2) konditioniert wird, um die Oberfläche des zu trocknenden Zerkleinerungsprodukts durch Ausbreiten in einer dünnen Schicht zu vergrößern, was zu einem Film mit einer Stärke zwischen 0,5 und 5 cm führt, oder auch durch Formgebung vom Typ Extrusion oder Durchleitung durch eine Düse, die erlaubt, die Verdunstungsoberfläche zu vergrößern.

**7.** Verfahren zur Gewinnung eines Avocadoöls nach einem der Ansprüche 1 bis 6, **dadurch gekennzeichnet, dass** das Trocknen (2) des Zerkleinerungsprodukts während 8 bis 78 Stunden durchgeführt wird.

**8.** Verfahren zur Gewinnung eines Avocadoöls nach Anspruch 7, **dadurch gekennzeichnet, dass** das Trocknen (2) des Zerkleinerungsprodukts während 10 bis 24 Stunden durchgeführt wird.

**9.** Avocadoöl, das durch das Verfahren nach einem der Ansprüche 1 bis 8 gewinnbar ist, **dadurch gekennzeichnet,**

**dass** es mindestens 3 Ma% Unverseifbares in Bezug auf die Gesamtmasse des Öls und mindestens 2 Ma% aliphatische Acetogenine und/oder deren Derivate in Bezug auf die Gesamtmasse des Öls enthält, wobei die aliphatischen Acetogenine und deren Derivate polyhydroxylierte Fettalkohole und deren acetylierte Derivate und/oder 1,2-Dihydroxy-4-oxo-aliphatische Alkohole und deren acetylierte Derivate vom Typ "Persine" und/oder aliphatische Furane sind.

**10.** Avocadoöl nach Anspruch 9, das mindestens 5 Ma% Unverseifbares in Bezug auf die Gesamtmasse des Öls enthält und/oder mindestens 3 Ma% aliphatische Acetogenine und/oder deren Derivate in Bezug auf die Gesamtmasse des Öls enthält.

**11.** Avocadoöl nach Anspruch 9 oder 10, **dadurch gekennzeichnet, dass** es einen Säureindex von unter oder gleich 5 mgKOH/g aufweist.

**12.** Avocadoöl nach Anspruch 11, **dadurch gekennzeichnet, dass** es einen Säureindex von unter oder gleich 3 mg-KOH/g aufweist.

**13.** Avocadoöl nach Anspruch 11, **dadurch gekennzeichnet, dass** es einen Säureindex von unter oder gleich 1 mg-KOH/g aufweist.

**14.** Avocadoöl nach einem der Ansprüche 9 bis 13, das mindestens 3 Ma% Unverseifbares in Bezug auf die Gesamtmasse des Öls, Avocado-Unverseifbares reich an aliphatischen Furanen oder mit Unverseifbarem angereichertes Avocadoölkonzentrat enthält, wobei das Unverseifbare oder das Konzentrat aus Avocadoöl nach einem der Ansprüche 9 bis 13 für ihre Verwendung als Arzneimittel zur Vorbeugung und/oder Behandlung von Beschwerden des Bindegewebes wie Arthrose, Gelenkerkrankungen wie Rheuma oder auch Parodontalkrankheiten wie Gingivitis oder Parodontitis gewinnbar sind.

## Claims

**1.** A process for obtaining an avocado oil from whole soft avocados, said oil containing at least 3% unsaponifiable by mass relative to the total mass of the oil, in which said unsaponifiable contains aliphatic acetogenins and/or their derivatives, the soft avocados having a penetration resistance force in the flesh less than or equal to 3 kg/cm$^2$, said penetration resistance force being measured by means of a penetrometer, comprising the following successive steps:

(1) Grinding of the soft avocados resulting in obtaining a ground product granulometry of between 2 and 20 mm,
(2) Drying of the ground product at high temperature between 60 and 150°C, to obtain a residual humidity less than or equal to 5%,
(3) Adding water to the dried avocados by addition of 1 to 5% of water or steam relative to the mass of ground dry avocados, then
(4) Extraction of the oil by mechanical pressure.

**2.** The process for obtaining avocado oil as claimed in Claim 1, **characterised in that** the grinding (1) is carried out on the whole avocados constituted by the skin, the pulp and the kernel, by shredding of the skin, grinding of the kernel and blending of the mixture to obtain a homogeneous dispersion of the ground product, advantageously by means of a grinder of serrated knives or rollers type.

**3.** The process for obtaining an avocado oil as claimed in Claim 1, **characterised in that** the grinding (1) is carried out by:

- Separation of the kernel and of the skin from the pulp of the avocados,
- Grinding of the different parts of the soft avocados, then
- Blending of the different ground parts to obtain a homogeneous dispersion.

**4.** The process for obtaining an avocado oil as claimed in Claim 1, **characterised in that** the grinding (1) of the soft avocados results in obtaining a ground product granulometry of between 2 and 10 mm.

**5.** The process for obtaining an avocado oil as claimed in Claim 1, **characterised in that** the drying (2) of the ground product is carried out at high temperature between 70 and 100°C.

6. The process for obtaining an avocado oil as claimed in any one of claims 1 to 5, **characterised in that** following grinding (1) and prior to drying (2), conditioning of the ground product is carried out to boost the surface of the ground product to be dried, by spreading in a thin layer, to result in a film of a thickness of between 0.5 and 5 cm, or by shaping to optimise the evaporation surface of the extrusion type or passing through a die.

7. The process for obtaining an avocado oil as claimed in any one of Claims 1 to 6, **characterised in that** the drying (2) of the ground product is carried out for 8 to 78 h.

8. The process for obtaining an avocado oil as claimed in Claim 7, **characterised in that** the drying (2) of the ground product is carried out for 10 to 24 h.

9. Avocado oil obtainable by the process as claimed in any one of Claims 1 to 8, **characterised in that** it contains at least 3% of unsaponifiable by mass, relative to the total mass of the oil and at least 2% by mass of aliphatic acetogenins and/or of their derivatives, relative to the total mass of the oil, said aliphatic acetogenins and their derivatives being fatty polyhydroxy alcohols and their acetylated derivatives, and/or 1,2-dihydroxy-4-oxo-aliphatic alcohols and their acetylated derivatives of "persin" type, and/or aliphatic furans.

10. The avocado oil as claimed in Claim 9, containing at least 5% of unsaponifiable mass, relative to the total mass of the oil, and/or containing at least 3% by mass of aliphatic acetogenins and/or of their derivatives, relative to the total mass of the oil.

11. The avocado oil as claimed in Claim 9 or 10, **characterised in that** it has an acid value less than or equal to 5 mgKOH/g.

12. The avocado oil as claimed in Claim 11, **characterised in that** it has an acid value less than or equal to 3 mgKOH/g.

13. The avocado oil as claimed in Claim 11, **characterised in that** it has an acid value less than or equal to 1 mgKOH/g.

14. The avocado oil as claimed in any one of Claims 9 to 13 containing at least 3% of unsaponifiable mass, relative to the total mass of the oil, an avocado unsaponifiable rich in aliphatic furans or a concentrate of avocado oil enriched in unsaponifiable, said unsaponifiable or said concentrate being obtainable from the avocado oil as defined in any one of Claims 9 to 13, for its use as a drug, advantageously in the prevention and/or the treatment of connective tissue disorders such as arthrosis, articular pathologies such as rheumatism, or periodontal disorders, such as gingivitis or periodontitis.